# FASCICULE DE BREVET EUROPEEN

(11) **EP 0 513 712 B1**
(45) Date de publication et mention de la délivrance du brevet: **24.01.1996**
(21) Numéro de dépôt: 92107894.5
(22) Date de dépôt: 11.05.1992
(51) Int. Cl.: A61B 5/087, A63B 23/18

(54) **Dispositif pour exercices respiratoires**
Gerät für Atemübungen
Pulmonary exercises device

(30) Priorité: 14.05.1991 CH 1435/91
(43) Date de publication de la demande: 19.11.1992
(73) Titulaire: Lugon, Guillermina, CH-1207 Genève (CH)
(72) Inventeur: Lugon, Georges, CH-1207 Genève (CH)
(74) Mandataire: Micheli & Cie

(56) Documents cités:
- FR-A- 2 222 074
- US-A- 3 977 395

## Description

Il existe des appareils destinés à faire des exercices respiratoires sous forme d'inspiration avec une résistance ou d'expiration contre une résistance. Pour faire des exercices respiratoires complets, inspiration et expiration, il faut utiliser deux appareils différents. Comme état de la technique le plus proche de la présente invention, on connait du document FR-A-2.222.074 un expirateur composé d'un récipient cylindrique, d'un couvercle muni d'un organe de réglage du débit et d'un manchon central ainsi que d'un tuyau barboteur. La présente invention a pour but la réalisation d'un appareil destiné à faire des exercices respiratoires pouvant fonctionner soit comme inspirateur avec une résistance variable, soit comme expirateur contre une résistance variable, de sorte qu'en couplant deux appareils semblables on obtient un respirateur dont les résistances peuvent être réglées indépendamment. Le dispositif selon l'invention se distingue par les caractéristiques énumérées à la revendication 1.

Les dessins annexés illustrent schématiquement et à titre d'exemple, une forme d'exécution du dispositif pour exercices respiratoires.

La figure 1 est une vue en coupe du dispositif monté en expirateur.

La figure 2 est une vue en coupe du dispositif monté en inspirateur.

La figure 3 est une vue en coupe de deux dispositifs montés en respirateur.

La figure 4 est une vue en coupe du boîtier contenant le clapet anti-retour.

La figure 5 est une vue de dessus du boîtier illustré à la figure 4.

La figure 6 est une vue en perspective du couvercle muni de son organe de réglage de débit dans la position fermée.

La figure 7 est une vue en perspective du couvercle muni de son organe de réglage de débit dans la position ouverte.

Le dispositif pour exercices respiratoires illustré à titre d'exemple est composé d'un récipient cylindrique transparent 1, d'un couvercle 2 comprenant un organe de contrôle du débit 3, d'un tuyau flexible 4, et d'un tube barboteur 5 terminé par un organe anti-retour 6.

Le récipient 1 d'une contenance d'un litre et demi est gradué de 100 en 100 millilitres, il comporte dans sa partie supérieure un filetage 7. Ce récipient est légèrement conique, le diamètre médian du récipient est égal au diamètre des trous pratiqués dans une plaque de fixation (non illustrée) décrite plus loin.

Le couvercle 2 comporte dans sa partie intérieure un pas de vis 8 permettant de fermer hermétiquement le récipient cylindrique 1.

Le couvercle présente une ouverture 9, située à mi distance entre le centre et le bord du couvercle, munie d'un manchon légèrement conique 10 destiné à recevoir l'organe de contrôle du débit. Le couvercle comporte encore une ouverture 11 en son centre dont le diamètre est sensiblement inférieur au diamètre du tuyau flexible 4. Cette ouverture est prolongée par un manchon cylindrique 12, légèrement conique, vers l'extérieur du couvercle permettant d'y introduire le tuyau flexible 4 et se prolongeant vers l'intérieur également sous la forme d'un manchon cylindrique 13 légèrement conique, destinée à recevoir le boîtier de l'organe anti-retour.

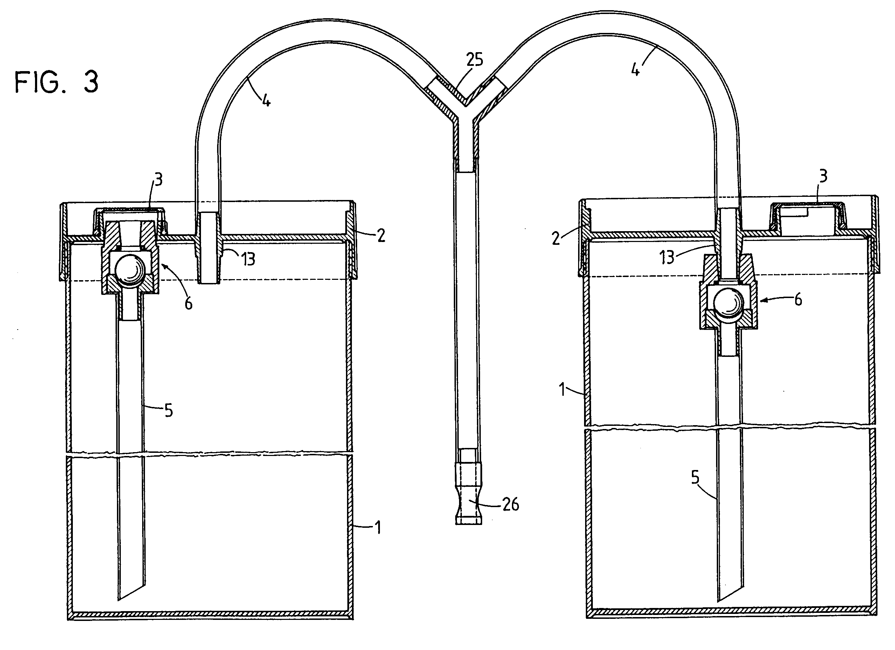

Le tuyau flexible 4 est raccordé sur le manchon cylindrique 12 et est terminé par un embout buccal 26 (illustré à la figure 3).

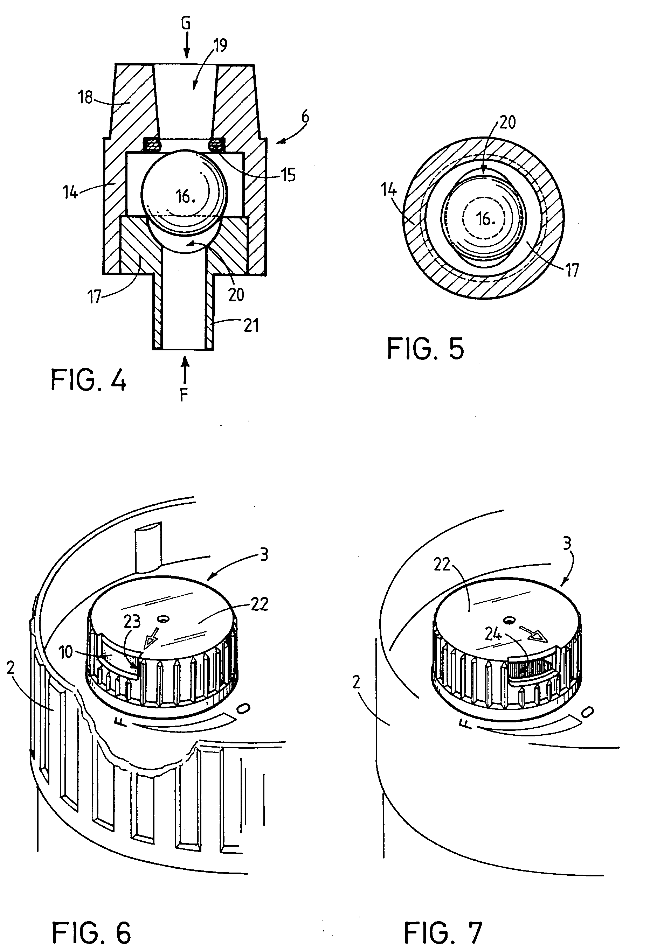

L'organe anti-retour, comme illustré à la figure 4, est composé d'un corps 14, d'un joint torique 15, d'une bille 16 et d'un embout 17. Le corps 14 est de forme cylindrique. Il présente une conicité 18 dans sa partie supérieure. La partie supérieure 18 du boîtier est conique en direction de l'extrémité supérieure de ce boîtier. Le diamètre externe de cette extrémité 18 du boîtier est légèrement inférieur au diamètre interne de l'ouverture 9 du couvercle. De cette façon le boîtier peut être fixé par emmanchement dans cette ouverture 9 réalisant une liaison étanche entre l'intérieur du couvercle et le tube barboteur 5. Cette partie supérieure 18 comporte également un trou tronconique 19 dont le plus grand diamètre est légèrement supérieur au diamètre extérieur du manchon cylindrique 13 du couvercle, ce qui permet de l'introduire sur le manchon cylindrique 13 du couvercle et d'assurer d'une part la fixation du boîtier 14 à ce manchon 13 et l'étanchéité entre le perçage 11 et le tube barboteur 5.

Un joint torique qui peut être réalisé en néoprène est logé dans un épaulement situé à l'extrémité inférieure du trou tronconique 19. Ce joint d'un diamètre inférieur à celui de la bille empêche le passage du flux d'air dans le sens de la flèche F lorsque l'appareil fonctionne en mode inspirateur. En effet tout flux d'air dans le sens de la flèche F applique la bille 16 contre le joint 15. La partie inférieure du corps comporte un chambrage de diamètre supérieur afin de pouvoir y introduire la bille 16 et recevoir l'embout 17.

L'embout 17 comporte une partie cylindrique d'un diamètre sensiblement égal au diamètre du chambrage inférieur du corps 14 ce qui permet de le rendre solidaire du corps 14 par emboîtage et éventuellement collage. L'embout 17 comporte un manchon 21 sur lequel vient se fixer le tuyau barboteur 5. Un évidement 20 de forme elliptique est pratiqué dans l'embout. Le grand diamètre de cette ellipse est supérieur au diamètre de la bille 16 ce qui permet au flux d'air de passer dans le sens de la flèche G malgré la présence de la bille lorsque l'appareil fonctionne en mode expirateur. Les dimensions de cet évidement 20 sont telles que la section de passage pour le flux d'air, la bille 16 reposant par gravité sur l'embout 17, soit au moins égale à la section du tube barboteur 5 ou de l'embout 21.

L'organe de réglage du débit 3, comme illustré à la figure 6, est composé d'un robinet 22 en forme de bouchon cylindrique dans lequel est pratiqué une ouverture rectangulaire 23. Le bouchon vient se loger sur le manchon 10 du couvercle également muni d'une ouverture rectangulaire 24. En tournant le bouchon cylindrique de façon à faire coïncider les deux ouvertures 23 et 24 on modifie la section de l'ouverture et on règle ainsi le débit d'air. Le manchon 10 du couvercle peut être muni en plus de l'organe de réglage du débit d'un minimanomètre simple (non illustré), monté sur le bouchon 22, et indiquant la pression à l'expiration ainsi que la dépression à l'inspiration. L'organe de réglage du débit 3 peut également être muni d'un obturateur rotatif mobile (non illustré) asservi par un mini moteur à piles et qui permet d'obturer séquentiellement l'ouverture 24, ceci dans le but de modifier le flux linéaire en un flux ondulatoire aussi bien à l'expiration qu'à l'inspiration.

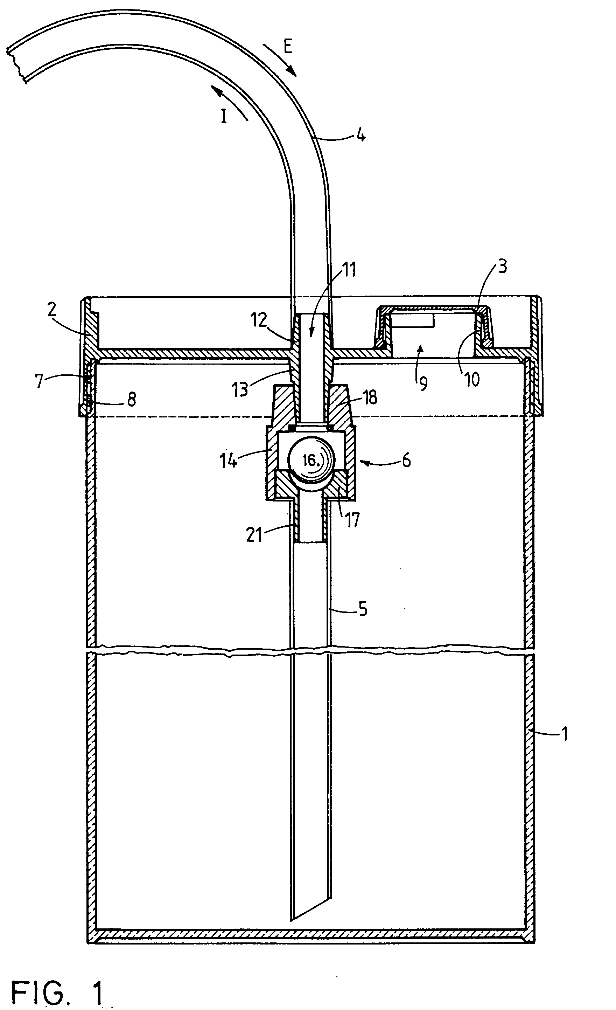

La figure 1 montre l'appareil fonctionnant en mode expirateur, on emboîte le corps 14 de l'organe anti-retour sur le manchon cylindrique 13 du couvercle. Dans ce cas le flux d'air ne peut aller que dans le sens de l'expiration (flèche E). En réglant l'ouverture de l'organe de contrôle du débit on augmente ou diminue la résistance. Pour augmenter la résistance on peut soit fermer davantage le robinet 22 soit augmenter le volume d'eau contenu dans le récipient cylindrique 1.

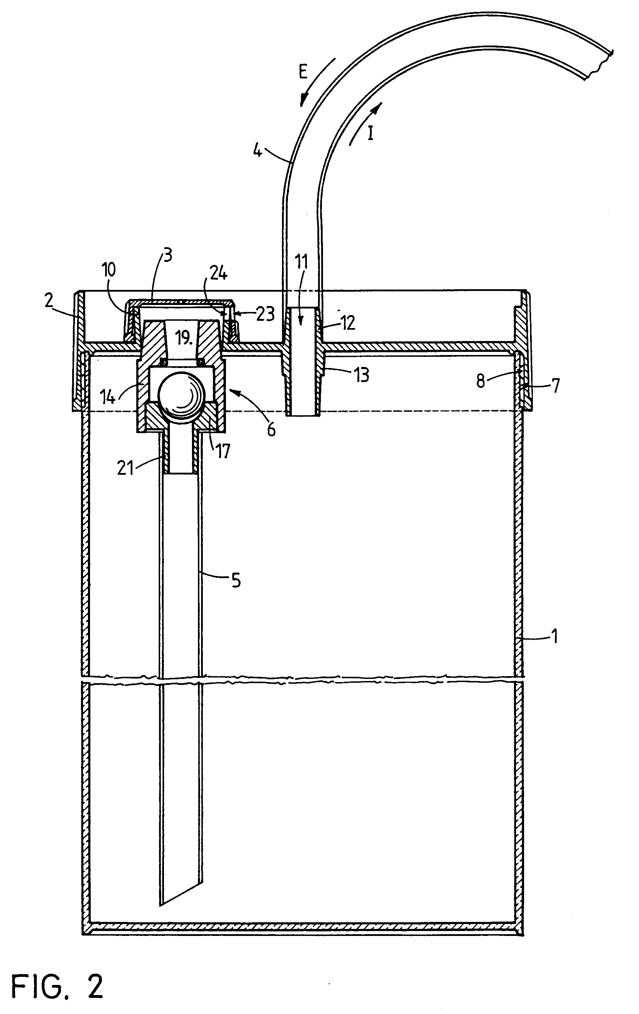

La figure 2 montre l'appareil fonctionnant en mode inspirateur, le corps 14 de l'organe anti-retour est enfiché dans le manchon 10 du couvercle. Le flux d'air ne peut aller que dans le sens de l'inspiration (flèche I). L'augmentation de la résistance à l'inspiration est obtenue comme précédemment, soit en fermant davantage le robinet 22 soit en augmentant le volume d'eau contenu dans le récipient.

La figure 3 montre comment brancher deux appareils identiques, l'un monté en inspirateur et l'autre en expirateur. On raccorde les tuyaux flexibles 4 à l'aide d'un raccord en Y 25. On obtient ainsi un appareil respirateur avec lequel on peut régler les résistances de façon indépendante.

Il est également prévu dans cette utilisation un support en matière plastique (non illustré) formé par une plaque rectangulaire percée de deux trous dans lesquels viennent se loger les récipients cylindriques 1 pour rendre l'ensemble solidaire et facilement transportable. Le diamètre des trous de la plaque correspond au diamètre extérieur de la partie médiane de la paroi des récipients qui est légèrement conique. La conicité nécessaire au démoulage des récipients 1 est suffisante pour qu'ils puissent être introduits dans le trou de la plaque par leurs extrémités inférieures et qu'ils soient maintenus dans lesdits trous.

Un tel dispositif est particulièrement avantageux car avec des éléments identiques, et donc une fabrication rationnelle on peut le monter soit en inspirateur soit en expirateur. De plus deux dispositifs identiques permettent la réalisation d'un respirateur complet.

## Revendications

1. Dispositif pour exercices respiratoires composé d'un récipient cylindrique (1), d'un couvercle (2) muni d'un organe de réglage du débit (3,9,10) et d'un manchon central (13), et d'un tuyau barboteur (5) caractérisé par le fait qu'il comporte un organe anti-retour (6) conformé de manière à pouvoir être fixé sur le manchon central (13) ou dans l'organe de réglage du débit (3,9,10), de sorte que l'appareil fonctionne en mode inspirateur ou en mode expirateur suivant la position dudit organe anti-retour (6).

2. Dispositif pour exercices respiratoires selon la revendication 1 caractérisé par le fait que le corps cylindrique (14) de l'organe anti-retour (6) est tronconique dans sa partie supérieure (18), la section de cette partie allant en diminuant vers l'extrémité libre du corps (14) et présentant un diamètre tel qu'il puisse être emmanché dans l'ouverture (9) excentrée du couvercle (2), par le fait que cette partie supérieure (18) est percée d'un canal allant en s'évasant vers l'extrémité libre du corps (14) dont les dimensions sont telles que le corps puisse être emmanché sur le manchon central (13) du couvercle (2).

3. Dispositif pour exercices respiratoires selon la revendication 1 caractérisé par le fait que l'organe de réglage du débit (3,9,10) comporte un minimanomètre fixé sur le bouchon cylindrique (3) dudit organe.

4. Dispositif pour exercices respiratoires selon la revendication 1 caractérisé par le fait que l'organe de réglage du débit (3,9,10) comporte un obturateur rotatif mobile (3) permettant de fermer séquentiellement l'ouverture (24) pratiquée dans le manchon (10) destiné à recevoir ledit organe de réglage du débit, de sorte que l'on obtienne un flux d'air ondulatoire.

5. Utilisation particulière de deux dispositifs pour entraîner la respiration selon la revendication 1 caractérisée par le fait que les ouvertures centrales (11) des couvercles (2) sont raccordées; que l'organe anti-retour (6) du premier dispositif est fixé sur le manchon central (13), tandis que l'organe anti-retour (6) du deuxième dispositif est fixé dans le manchon (10) de l'organe de contrôle du débit (3,9,10), de sorte que l'on obtienne un respirateur fonctionnant comme inspirateur et expirateur.

## Claims

1. A device for respiration exercises comprised of a cylindrical container (1), of a lid (2) provided with a flow controlling member (3, 9, 10), a central sleeve (13), and a bubbler tube (5), characterized in that it includes a nonreturn member (6) conformed in such a manner that it can be fastened on the central sleeve (13) or into the flow controlling member (3, 9, 10), so that the apparatus functions in the inspiration mode or in the expiration mode, according to the position of said nonreturn member (6).

2. A device for respiration exercises according to claim 1, characterized in that the cylindrical body (14) of the nonreturn member (6) is truncated in its upper part (18), the cross-section of this part decreasing towards the free end of the body (14) and exhibiting a diameter such that it can be inserted into the out of center opening (9) of the lid (2), and in that this upper part (18) has a channel extending therethrough of a diameter increasing towards the free end of the body (14), having dimensions such that the body may be fitted on the central sleeve (13) of the lid (2).

3. A device for respiration exercises according to claim 1, characterized in that the flow controlling member (3, 9, 10) includes a mini-manometer fastened to the cylindrical cap (3) of said member.

4. A device for respiration exercises according to claim 1, characterized in that the flow controlling member (3, 9, 10) includes a mobile rotatory shutter (3), which makes it possible to close repeatedly the opening (24) of the sleeve (10) designed for receiving said flow controlling device, so as to obtain an undulating flow of air.

5. The special use of two devices for respiration exercises according to claim 1, characterized in that the central openings (11) of the lids (2) are connected together; in that the nonreturn member (6) of the first device is fastened to the central sleeve (13), while the nonreturn member (6) of the second device is fastened to the sleeve (10) of the flow controlling member (3, 9, 10), so as to obtain a respirator functioning as an inspirator and an expirator.

## Patentansprüche

1. Vorrichtung für Atemübungen, bestehend aus einem zylindrischen Behältnis (1), einem mit einem Durchflussregelorgan (3, 9, 10) und einem zentralen Stutzen (13) versehenen Deckel (2) und einem Tauchrohr (5), dadurch gekennzeichnet, dass sie ein Rückschlagventil (6) umfasst, das so gestaltet ist, dass es auf dem zentralen Stutzen (13) oder im Durchflussregelorgan (3, 9, 10) befestigt werden kann, so dass das Gerät je nach der Stellung des benannten Rückschlagventils (6) als Einatem- oder Ausatem-Gerät funktioniert.

2. Vorrichtung für Atemübungen gemäss Anspruch 1, dadurch gekennzeichnet, dass der zylindrische Körper (14) des Rückschlagventils (6) in seinem oberen Abschnitt (18) kegelstumpfförmig ist, wobei sich der Querschnitt dieses Abschnitts zum freien Ende des Körpers (14) hin verjüngt und einen Durchmesser darbietet, der ein Einstecken in die exzentrische Öffnung (9) des Deckels gestattet; und dass durch diesen oberen Abschnitt (18) ein Kanal führt, der sich nach dem freien Ende des Körpers (14) hin ausweitet und dessen Abmessungen es gestatten, den Körper auf den zentralen Stutzen (13) des Deckels (2) aufzustecken.

3. Vorrichtung für Atemübungen gemäss Anspruch 1, dadurch gekennzeichnet, dass das Durchflussregelorgan (3, 9, 10) ein auf dem zylindrischen Ansatz (3) des benannten Organs befestigtes Minimanometer umfasst.

4. Vorrichtung für Atemübungen gemäss Anspruch 1, dadurch gekennzeichnet, dass das Durchflussregelorgan (3, 9, 10) einen beweglichen Drehverschluss (3) umfasst, der es gestattet, sequentiell die Öffnung (24) zu verschliessen, mit der der Stutzen (10) versehen ist, der dazu bestimmt ist, das benannte Durchflussregelorgan aufzunehmen, so dass man einen wellenartigen Luftstrom erhält.

5. Spezifische Benutzung zweier Atemtrainingsvorrichtungen gemäss Anspruch 1, dadurch gekennzeichnet, dass die zentralen Öffnungen (11) der Deckel (2) miteinander verbunden sind und dass das Rückschlagventil (6) der ersten Vorrichtung auf dem zentralen Stutzen (13) befestigt ist, während das Rückschlagventil (6) der zweiten Vorrichtung auf dem Stutzen (10) des Durchflussregelorgans (3, 9, 10) befestigt ist, so dass man ein Atemgerät erhält, das als Einatem- und Ausatem-Gerät wirkt.
